(19) Europäisches Patentamt
European Patent Office
Office européen des brevets

(11) **EP 4 088 677 A1**

(12) **EUROPÄISCHE PATENTANMELDUNG**

(43) Veröffentlichungstag:
**16.11.2022   Patentblatt 2022/46**

(21) Anmeldenummer: **22171604.6**

(22) Anmeldetag: **04.05.2022**

(51) Internationale Patentklassifikation (IPC):
**A61B 18/26** (2006.01)   **G01B 11/02** (2006.01)
**A61B 90/00** (2016.01)   **A61B 18/00** (2006.01)

(52) Gemeinsame Patentklassifikation (CPC):
**A61B 18/26; G01B 11/026;** A61B 2017/00066;
A61B 2018/00642; A61B 2018/00672;
A61B 2018/00678; A61B 2018/00702;
A61B 2018/00785; A61B 2090/061

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(30) Priorität: **12.05.2021   DE 102021112411**

(71) Anmelder: **Karl Storz SE & Co. KG**
**78532 Tuttlingen (DE)**

(72) Erfinder:
• **Brinkmann, Ralf**
**23562 Lübeck (DE)**
• **Lange, Birgit**
**23562 Lübeck (DE)**

(54) **ABSTANDSMESSVERFAHREN UND -VORRICHTUNG, SOWIE LASER-LITHOTRIPSIE-VORRICHTUNG**

(57)     Es wird ein Messverfahren zum optischen Bestimmen eines Abstands (z) einer in einem Medium (16) befindlichen Oberfläche (12) von einem Ende (18) eines optischen Wellenleiters (20) beschrieben, das folgende Schritte aufweist: Aussenden elektromagnetischer Messstrahlung (24, 26) einer ersten Wellenlänge ($\lambda$1) und einer zweiten Wellenlänge ($\lambda$2) von dem Ende (18) des Wellenleiters (20) zu der Oberfläche (12), wobei das Medium (16) die elektromagnetische Messstrahlung (26) der zweiten Wellenlänge ($\lambda$2) stärker absorbiert als die elektromagnetische Messstrahlung (24) der ersten Wellenlänge ($\lambda$1); Messen eines ersten Reflexionssignals ($I_1$) der von der Oberfläche (12) reflektierten elektromagnetischen Messstrahlung (24r) der ersten Wellenlänge ($\lambda$1), und Messen eines zweiten Reflexionssignals ($I_2$) der von der Oberfläche (12) reflektierten elektromagnetischen Messstrahlung (26r) der zweiten Wellenlänge ($\lambda$2), und Bestimmen des Abstandes (z) aus einem Verhältnis ($I_2/I_1$) aus dem zweiten und dem ersten Reflexionssignal. Des Weiteren wird eine Messvorrichtung und eine Laser-Lithotripsie-Vorrichtung beschrieben. (Fig. 1)

Fig. 1

**Beschreibung**

Die Erfindung betrifft ein Messverfahren zum optischen Bestimmen eines Abstands einer in einem Medium befindlichen Oberfläche eines Körpersteins von einem Ende eines optischen Wellenleiters.

Die Erfindung betrifft des Weiteren eine Messvorrichtung zum optischen Bestimmen eines Abstands einer in einem Medium befindlichen Oberfläche eines Körpersteins von einem Ende eines optischen Wellenleiters.

Schließlich betrifft die Erfindung eine Laser-Lithotripsie-Vorrichtung zum Zerkleinern von Körpersteinen.

Die Laser-Lithotripsie ist eine etablierte Form der Behandlung von Körpersteinen, beispielsweise von Steinen in der Blase oder im Harnleiter. Bei der Laser-Lithotripsie wird zur Zerkleinerung von Steinen Laserlicht über einen optischen Wellenleiter, bspw. eine optische Faser, auf den zu zerkleinernden Stein gerichtet. Zur Zerkleinerung der Steine werden verschiedene Techniken eingesetzt, die sich in Pulswiederholrate und Pulsenergie der Laserstrahlung sowie Positionierung und Bewegung des optischen Wellenleiters unterscheiden. Grundsätzlich ist die Zerkleinerung am effektivsten, wenn sich das Ende des optischen Wellenleiters, aus dem das Laserlicht austritt, in Kontakt mit dem oder sehr nahe am Stein befindet.

Fast alle humanen Körpersteine emittieren bei Anregung mit grünem Licht ein Autofluoreszenzsignal. Dieses Autofluoreszenzsignal kann während der Laser-Lithotripsie für eine automatische Steinerkennung genutzt werden, wie bspw. in dem Artikel von Lange B., Cordes J., Brinkmann R.: "Stone/tissue differentiation for holmium laser lithotripsy using autofluorescence"; Lasers Surg Med 2015; 47(9): 737-744, beschrieben ist. Bei bisherigen technischen Realisierungen eines automatisch geregelten Holmium-Lasersystems wird der Laser nur ausgelöst, wenn das Fluoreszenzsignal einen vorher festgelegten Schwellenwert überschritten hat (siehe Schlager D., Miernik A., Lamrini S. et al.: "A novel laser lithotripsy system with automatic real-time urinary stone recognition: Computer controlled ex vivo lithotripsy is feasible and reproducible in endoscopic stone fragmentation"; Journal of Urology 2019; 202: 1263-1269). Hierbei hat sich allerdings das Problem herausgestellt, dass die Intensität der Fluoreszenz verschiedener Steine unterschiedlich stark ist, wodurch der Abstand zwischen dem Ende des optischen Wellenleiters und dem Stein bei Überschreitung dieses Schwellenwertes nachteiligerweise um mehrere Millimeter variieren kann.

Es wurde bereits vorgeschlagen, in den Aufbau zur Steinerkennung mittels Fluoreszenzmessung eine Reflexionsmessung zu integrieren, wie in dem Artikel von Lange B., Cordes J., Brinkmann R.: "Exploiting the aiming beam to increase the safety of laser lithotripsy: Experimental evaluation of light reflection and fluorescence"; Lasers Surg Med 2020; 52(5); 456-471, beschrieben ist. Befindet sich keine Oberfläche vor dem Austrittsende der Behandlungsfaser, lassen sich aus dem Reflexionssignal Rückschlüsse auf den Zustand der Faserendfläche ziehen. Oberflächen vor der Faser führen zu einer abstandsabhängigen Signalerhöhung. Diese variiert jedoch nachteiligerweise mit der Oberflächenstruktur und dem Reflexionsgrad der Oberfläche des jeweiligen Steins, so dass keine hinreichend genaue Abstandsmessung ermöglicht wird.

Wenn das Ende des optischen Wellenleiters, mit dem das Behandlungslaserlicht zugeführt wird, von dem zu zerkleinernden Stein zu weit beabstandet ist, ist nicht nur die Wirkung des Behandlungslaserlichts in Bezug auf die Steinzerkleinerung unerwünscht verringert, was bei nicht ausreichendem Spülfluss zu einem signifikanten Temperaturanstieg im Behandlungsvolumen führen kann, sondern es besteht auch die Gefahr, dass das Behandlungslaserlicht unbeteiligtes Gewebe trifft und dieses schädigt.

Der Abstand des Austrittsendes des Wellenleiters, über das das Behandlungslaserlicht ausgesendet wird, ist somit ein wichtiger Betriebsparameter der Laser-Lithotripsie-Vorrichtung.

Der Erfindung liegt die Aufgabe zugrunde, ein Messverfahren und eine Messvorrichtung bereitzustellen, mit dem dieser Betriebsparameter mit höherer Genauigkeit bestimmt werden kann.

Der Erfindung liegt des Weiteren die Aufgabe zugrunde, eine verbesserte Laser-Lithotripsie-Vorrichtung bereitzustellen.

Erfindungsgemäß wird ein Messverfahren zum optischen Bestimmen eines Abstands einer in einem Medium befindlichen Oberfläche eines Körpersteins von einem Ende eines optischen Wellenleiters bereitgestellt, mit den Schritten:

Aussenden elektromagnetischer Messstrahlung einer ersten Wellenlänge und einer zweiten Wellenlänge von dem Ende des Wellenleiters zu der Oberfläche, wobei das Medium die elektromagnetische Messstrahlung der zweiten Wellenlänge stärker absorbiert als die elektromagnetische Messstrahlung der ersten Wellenlänge;

Messen eines ersten Reflexionssignals der von der Oberfläche reflektierten elektromagnetischen Messstrahlung der ersten Wellenlänge, und Messen eines zweiten Reflexionssignals der von der Oberfläche reflektierten elektromagnetischen Messstrahlung der zweiten Wellenlänge, und

Bestimmen des Abstandes aus einem Verhältnis aus dem zweiten und dem ersten Reflexionssignal.

Erfindungsgemäß wird des Weiteren eine Messvorrichtung zum optischen Bestimmen eines Abstands einer in einem Medium befindlichen Oberfläche eines Körpersteins von einem Ende eines optischen Wellenleiters bereitgestellt, mit:

einer Messstrahlungsquelle zum Erzeugen elektromagnetischer Messstrahlung einer ersten Wellenlänge und einer zweiten Wellenlänge, wobei das Medium die elektromagnetische Messstrahlung der zweiten Wellenlänge stärker absorbiert als die elektromagnetische Messstrahlung der ersten Wellenlänge,

einem optischen Wellenleiter zum Aussenden der elektromagnetischen Messstrahlung vom Ende des Wellenleiters zu der Oberfläche;

einer Detektionsvorrichtung zum Messen eines ersten Reflexionssignals der von der Oberfläche reflektierten elektromagnetischen Messstrahlung der ersten Wellenlänge, und zum Messen eines zweiten Reflexionssignals der von der Oberfläche reflektierten elektromagnetischen Messstrahlung der zweiten Wellenlänge, und

einer Auswerteeinheit zum Bestimmen des Abstandes aus einem Verhältnis aus dem zweiten und dem ersten Reflexionssignal.

Mit dem erfindungsgemäßen Messverfahren und der erfindungsgemäßen Messvorrichtung kann der Abstand eines Endes eines optischen Wellenleiters von der Oberfläche eines Zielobjekts zuverlässig bestimmt werden. Der Wellenleiter kann insbesondere eine optische Faser, insbesondere eine Multimodenfaser sein. Erfindungsgemäß wird elektromagnetische Messstrahlung mit zumindest zwei unterschiedlichen Wellenlängen vom Ende des Wellenleiters auf die Oberfläche des Objekts gerichtet. Die erste und die zweite Wellenlänge sind so gewählt, dass die Messstrahlung der einen Wellenlänge von dem Medium stärker absorbiert wird als die Messstrahlung der anderen Wellenlänge. Die gemessenen Reflexionssignale zeigen somit eine unterschiedliche Abhängigkeit vom Abstand des Endes des optischen Wellenleiters von der Oberfläche des Zielobjekts, an der die Messstrahlung reflektiert wird. Mit anderen Worten fällt das Reflexionssignal der Messstrahlung der Wellenlänge, die stärker absorbiert wird, als Funktion des Abstands schneller ab als das Reflexionssignal der anderen Wellenlänge.

Der Begriff "Reflexion" umfasst im Sinne der vorliegenden Erfindung nicht nur gerichtete, d.h. spiegelnde Reflexion, sondern auch diffuse, d.h. zerstreute Reflexion. Durch die Nutzung von Messstrahlung zumindest zweier Wellenlängen mit unterschiedlicher Absorption und die Verhältnisbildung aus den zugehörigen Reflexionssignalen wirken sich unterschiedliche Reflexionsgrade unterschiedlicher Oberflächen auf das Messergebnis der Abstandsmessung nicht oder deutlich verringert negativ aus, insbesondere wenn die Reflexionsgrade nicht oder nur schwach wellenlängenabhängig sind, zumindest aber deutlich weniger von der Wellenlänge abhängen als die Absorption im Medium. Wenn dagegen die Abstandsmessung mit nur einer Wellenlänge durchgeführt wird, ist es nicht möglich, aus der Intensität des Reflexionssignals auf den Abstand zwischen der Oberfläche und dem Ende des optischen Wellenleiters zu schließen, da das Reflexionsvermögen von Oberflächen verschiedener Körper, und dies gilt auch für Körpersteine, stark variieren kann.

Das Medium kann insbesondere ein flüssiges Medium sein, beispielsweise Wasser oder eine wässrige Lösung, oder auch ein gasförmiges Medium, das bei unterschiedlichen Wellenlängen unterschiedlich stark absorbiert.

Das erfindungsgemäße Messverfahren bzw. die erfindungsgemäße Messvorrichtung kann insbesondere zum Bestimmen des Betriebsparameters ‚Abstand eines Endes einer Behandlungsfaser von der Oberfläche eines Steins' in einer Laser-Lithotripsie-Vorrichtung verwendet werden. Es versteht sich allerdings, dass das erfindungsgemäße Messverfahren nicht auf eine Anwendung in der Laser-Lithotripsie beschränkt ist, sondern allgemein in der Messtechnik zur Abstandsmessung eingesetzt werden kann.

Das erfindungsgemäße Messverfahren eignet sich insbesondere zur Bestimmung kleiner Abstände im Bereich von wenigen Zehntel Millimetern bis einigen Millimetern.

Die erste Wellenlänge kann so gewählt werden, dass sie von dem Medium nicht oder nur schwach absorbiert wird, während die zweite Wellenlänge vorzugsweise so gewählt wird, dass aufgrund der stärkeren Absorption das zweite Reflexionssignal innerhalb eines gewünschten Abstandsbereichs auf weniger als einen Bruchteil der maximal messbaren Intensität abfällt.

Das Bestimmen des Abstandes kann in einer bevorzugten Ausführungsform ein Bestimmen umfassen, ob ein vorbestimmter Maximalabstand unterschritten ist. Für manche Anwendungen, beispielsweise in der Laser-Lithotripsie, ist es nicht erforderlich, den genauen Wert des gemessenen Abstands zu kennen, sondern lediglich, ob der gemessene Abstand unterhalb eines Grenzwertes liegt. Das Auswerten der Reflexionssignale kann hierdurch vereinfacht werden. Alternativ oder kumulativ zu der vorstehenden Maßnahme kann das Bestimmen des Abstandes in einer bevorzugten Ausführungsform ein Bestimmen umfassen, ob ein vorbestimmter Mindestabstand überschritten ist. Dies kann vorteilhafterweise dazu dienen zu vermeiden, dass das Ende des Wellenleiters mit der Oberfläche in Kontakt kommt. Das Ende oder die Endfläche des Wellenleiters kann somit geschont werden.

Die Auswerteeinheit kann ein Triggersignal erzeugen, wenn die Messung ergibt, dass der vorbestimmte Maximalabstand unterschritten und/oder der vorbestimmte Mindestabstand überschritten ist, bzw. wenn die Messung ergibt, dass der gemessene Abstand zwischen dem Mindestabstand und dem Maximalabstand liegt. Ein solches Triggersignal kann eine Aktivierung einer Vorrichtung oder eine Freischaltung einer Vorrichtung zur Aktivierung derselben auslösen. Beispielsweise im Rahmen der Laser-Lithotripsie kann das Triggersignal den Behandlungslaser freischalten, um ihn dann aktivieren zu können.

Vorzugsweise wird die zweite Wellenlänge so gewählt, dass das zweite Reflexionssignal in einem Abstand von dem Ende des Wellenleiters unterhalb des vorbestimmten Maximalabstandes auf einen Bruchteil von weniger als 20%, vorzugsweise von weniger als 10%, weiter vorzugsweise von weniger als 5% der maximal messbaren Intensität abfällt.

Mit anderen Worten kann die zweite Wellenlänge so gewählt sein, dass nur Abstände detektiert werden, die kleiner sind als ein vorbestimmter Abstandsgrenzwert, während bei Abständen, die größer sind als der Abstandsgrenzwert, das zweite Reflexionssignal so schwach sein kann, dass es sich von einem Rauschen nicht unterscheidet. Wird kein zweites Reflexionssignal gemessen, bedeutet dies somit, dass der Abstand des Endes des Wellenleiters von der Oberfläche des Zielobjekts außerhalb des gewünschten Abstandsbereichs liegt.

In einer weiteren bevorzugten Ausgestaltung kann in Abhängigkeit des vorbestimmten Maximalabstandes ein erster Schwellenwert für das zweite Reflexionssignal vorbestimmt werden, und das Verhältnis aus dem zweiten Reflexionssignal und dem ersten Reflexionssignal kann mit 1 multipliziert werden, wenn das zweite Reflexionssignal den Schwellenwert für das zweite Reflexionssignal überschreitet, und kann ansonsten auf null gesetzt werden, wobei in Abhängigkeit des vorbestimmten Maximalabstandes ein zweiter Schwellenwert für das Verhältnis aus dem zweiten Reflexionssignal und dem ersten Reflexionssignal vorbestimmt werden kann.

Diese Maßnahme ist für eine praktische Implementierung des erfindungsgemäßen Messverfahrens und der Messvorrichtung vorteilhaft, wenn, wie bspw. bei der Laser-Lithotripsie, nur Abstände unterhalb eines Maximalabstands von Interesse sind. Mit dieser Maßnahme wird außerdem mit einer einfachen Rechenoperation, nämlich der Multiplikation des Verhältnisses der Reflexionssignale mit einer Treppenfunktion, die Werte 0 und 1 hat, eine einfache Auswertung der Reflexionssignale ermöglicht, mit der Abstände unterhalb eines gewünschten Maximalabstands verlässlich detektiert werden können.

In einer weiteren bevorzugten Ausführungsform kann die zweite Wellenlänge so gewählt sein, dass sich der Absorptionskoeffizient des Mediums bei der zweiten Wellenlänge von dem Absorptionskoeffizient bei der ersten Wellenlänge um einen Faktor von zumindest 100, vorzugsweise von zumindest 1000, weiter vorzugsweise von zumindest 10000 unterscheidet.

Je stärker die Absorption der Messstrahlung der zweiten Wellenlänge durch das Medium ist, desto schneller fällt das zweite Reflexionssignal mit zunehmendem Abstand vom Ende des Wellenleiters ab. Ein starker Abfall des zweiten Reflexionssignals ist insbesondere bei der Messung sehr kleiner Abstände von weniger als 1 mm vorteilhaft.

In weiteren bevorzugten Ausführungsformen kann die erste Wellenlänge im sichtbaren Spektralbereich liegen, und/oder die zweite Wellenlänge im Nah-Infrarot-Spektralbereich.

Die erste Wellenlänge kann bspw. so gewählt sein, dass sie das Zielobjekt zur Autofluoreszenz anregt, wie dies bereits bei den herkömmlichen Techniken zur Körpersteinerkennung verwendet wurde. Die erste Wellenlänge kann somit zur Differenzierung zwischen Steinen und Gewebe genutzt werden Die erste Wellenlänge kann bspw. im grünen Spektralbereich liegen. Wenn die zweite Wellenlänge im Nah-Infrarot-Spektralbereich liegt, wird die Messstrahlung der zweiten Wellenlänge sehr stark absorbiert. Beispielsweise bei einer Wellenlänge von 1310 nm kann das Reflexionssignal eines Spiegels in Wasser, je nach numerischer Apertur des Wellenleiters und Einstrahlwinkel auf den Spiegel, innerhalb von 1 mm auf etwa 4 % des Maximalwertes des Signals am Ende des Wellenleiters abfallen.

Gemäß einem weiteren Aspekt der Erfindung kann alternativ oder zusätzlich zur Wahl der zweiten Wellenlänge derart, dass der Absorptionskoeffizient des Mediums bei dieser Wellenlänge höher ist als bei der ersten Wellenlänge, in einer bevorzugten Ausführungsform die Messstrahlung der ersten Wellenlänge mit einem ersten Öffnungswinkel in den Wellenleiter eingekoppelt werden, der sich von einem Öffnungswinkel unterscheidet, mit dem die Messstrahlung der zweiten Wellenlänge in den Wellenleiter eingekoppelt wird.

Des Weiteren kann im Rahmen eines noch weiteren Aspekts der Erfindung die Messstrahlung der ersten oder der zweiten Wellenlänge schräg zur Wellenleiterlängsachse in den Wellenleiter eingekoppelt werden.

Mit den beiden vorstehend genannten Maßnahmen, die auch in Kombination miteinander verwendet werden können, kann ebenfalls erreicht werden, dass eines der Reflexionssignale als Funktion des Abstandes vom Ende des Wellenleiters schneller abfällt als das andere Reflexionssignal, indem die Messstrahlungen unterschiedlich in den Wellenleiter eingekoppelt werden. Mit anderen Worten kann durch unterschiedliche Nutzung der numerischen Apertur des Wellenleiters und/oder durch eine ‚schiefe' Einkopplung der Messstrahlung das Abstandsverhalten der Reflexionssignale beeinflusst werden. Die unterschiedlichen Einkopplungen der Messstrahlungen ergeben unterschiedliche Strahlprofile am Ausgang des Wellenleiters, die nach Reflexion an der Oberfläche des Zielobjektes zu unterschiedlichen Abstandsverhalten der Reflexionssignale führen. Mit einer schiefen Einkopplung kann beispielsweise ausgangsseitig des Wellenleiters ein Donut-förmiges Strahlprofil erzeugt werden. Diese Maßnahmen können insbesondere dann vorteilhaft sein, wenn, bspw. keine Messstrahlenquelle verfügbar ist, die Messstrahlung einer Wellenlänge bereitstellen kann, die von dem Medium im gewünschten Maß absorbiert wird. So können im Rahmen dieses Aspektes die erste Wellenlänge und die zweite Wellenlänge sogar gleich sein.

Die Erfindung umfasst unabhängig von Anspruch 1 daher auch folgende Aspekte:

Messverfahren zum optischen Bestimmen eines Abstands einer in einem Medium befindlichen Oberfläche von einem Ende eines optischen Wellenleiters, mit den Schritten:

Aussenden einer ersten elektromagnetischen Messstrahlung und einer zweiten elektromagnetischen Messstrahlung vom Ende des Wellenleiters zu der Oberfläche, wobei die erste Messstrahlung mit einem ersten Öffnungswinkel in den Wellenleiter eingekoppelt wird, der sich von einem Öffnungswinkel unterscheidet, mit dem die zweite Messstrahlung in den Wellenleiter eingekoppelt wird, und/oder die erste Messstrahlung oder die zweite Messstrahlung wird schräg zur Wellenleiterlängsachse in den Wellenleiter eingekoppelt;

Messen eines ersten Reflexionssignals der von der Oberfläche reflektierten ersten elektromagnetischen Messstrahlung, und Messen eines zweiten Reflexionssignals der von der Oberfläche reflektierten zweiten elektromagnetischen Messstrahlung, und

Bestimmen des Abstandes aus einem Verhältnis aus dem zweiten und dem ersten Reflexionssignal.

Der optische Wellenleiter kann eine optische Faser, insbesondere eine Multimodenfaser sein.

Die Messstrahlungsquelle kann eine breitbandige Messstrahlungsquelle sein. Die erste Wellenlänge und die zweite Wellenlänge können durch der Messstrahlungsquelle nachgeschaltete Filter durchgelassen werden, während andere Spektralbereiche der erzeugten elektromagnetischen Messstrahlung blockiert werden.

Die Messstrahlungsquelle kann jedoch auch für die erste Wellenlänge, die zweite Wellenlänge und ggf. weitere Wellenlängen jeweils separate Lichtquellen aufweisen, insbesondere einen ersten Laser zum Erzeugen der elektromagnetischen Messstrahlung der ersten Wellenlänge und einen zweiten Laser zum Erzeugen der elektromagnetischen Messstrahlung der zweiten Wellenlänge, wie in einer bevorzugten Ausführungsform vorgesehen ist.

Die Detektionsvorrichtung kann einen Detektor aufweisen, der zum Messen der Reflexionssignale der ersten und der zweiten Wellenlänge geeignet ist, wobei dem Detektor entsprechende Spektralfilter vorgeschaltet sein können. Eine Separierung der beiden Wellenlängen am Detektor kann auch durch Zeit-Multiplexing erreicht werden. Ebenso ist es möglich, dass die Detektionsvorrichtung für jede Wellenlänge einen separaten Detektor aufweist.

Die Messstrahlungsquelle kann dazu ausgelegt sein, die erste Wellenlänge im sichtbaren Spektralbereich zu erzeugen, und/oder die zweite Wellenlänge im nahen Infrarot zu erzeugen.

Wie bereits oben beschrieben, kann die Einkopplung der Messstrahlung in den optischen Wellenleiter so erfolgen, dass die numerische Apertur des optischen Wellenleiters unterschiedlich ausgenutzt wird, d.h. die Messstrahlung mit unterschiedlichen Öffnungswinkeln in den Wellenleiter eingekoppelt wird. Dies kann durch eine den Lichtquellen nachgeschaltete Optik mit unterschiedlichen numerischen Aperturen realisiert sein. Alternativ oder kumulativ kann, wie bereits oben erwähnt, die von der Messstrahlungsquelle erzeugte erste oder zweite Messstrahlung schräg zur Wellenleiterlängsachse in den Wellenleiter eingekoppelt werden, was durch eine Optik oder durch eine schräge Anordnung des Eintrittsende des Wellenleiters relativ zur Emissionsrichtung der Messlichtquelle realisiert werden kann.

Der optische Wellenleiter weist vorzugsweise eine numerische Apertur von größer als 0,1, vorzugsweise größer als 0,2 auf.

Die Auswerteeinheit kann als Mikroprozessor realisiert sein, der in die Detektionsvorrichtung integriert sein kann. Die Auswerteeinheit kann aber auch als Software realisiert sein.

Weiter erfindungsgemäß wird eine Laser-Lithotripsie-Vorrichtung zum Zerkleinern von Körpersteinen bereitgestellt, mit einem Behandlungslaser zum Emittieren von Behandlungslaserlicht und einer Messvorrichtung nach einer oder mehreren der oben genannten Ausgestaltungen.

Vorzugsweise wird das Behandlungslaserlicht in denselben Wellenleiter eingespeist und von diesem abgegeben, mit dem auch die Abstandsmessung durchgeführt wird.

Der Behandlungslaser kann bspw. ein Holmium-Laser sein. Im Fall der Lithotripsie ist das Medium Harn- oder Spülflüssigkeit.

Die Auswerteeinheit der Messvorrichtung kann dazu ausgelegt ein, ein Triggersignal zum Freischalten des Behandlungslasers zu erzeugen, wenn ein Abstand des Endes des Wellenleiters von der Oberfläche eines zu zerkleinernden Körpersteins gemessen wird, der kleiner ist als ein vorbestimmter Maximalabstand. Der Behandlungslaser wird somit nur dann zur Aktivierung freigeschaltet, wenn sich das Ende des Wellenleiters nahe genug am Körperstein befindet. Zusätzlich wird das Triggersignal vorzugsweise nur erzeugt, wenn der gemessene Abstand des Endes des Wellenleiters von der Oberfläche des Körpersteins größer als ein Mindestabstand ist. In einer weiteren bevorzugten Ausgestaltung kann die Laser-Lithtripsie-Vorrichtung eine Steuerungseinrichtung für den Behandlungslaser aufweisen, die dazu ausgelegt ist, die Pulsenergie des Behandlungslaserlichtes in Abhängigkeit von dem gemessenen Abstand anzupassen. Beispielsweise kann die Steuerungseinrichtung die Pulsenergie erhöhen, wenn sich das Ende des Wellenleiters in größerem Abstand von der Oberfläche des Körpersteines befindet, bzw. bei kleinerem Abstand verringern. Die Effizienz der Behandlung kann hierdurch verbessert werden.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der Erfindung zu verlassen.

Ausführungsbeispiele der Erfindung sind in der Zeichnung dargestellt und werden mit Bezug auf diese hiernach näher beschrieben. Es zeigen:

Fig. 1            ein Blockschaltbild einer Messvorrichtung zum optischen Bestimmen eines Abstands einer in einem Medium befindlichen Oberfläche von einem Ende eines optischen Wellenleiters;

Fig. 2            ein Flussdiagramm eines Messverfahrens zum optischen Bestimmen eines Abstandes einer in einem Medium befindlichen Oberfläche von einem Ende eines optischen Wellenleiters;

Fig. 3a)          ein Diagramm, das Reflexionssignale von Oberflächen verschiedener Zielobjekte in Abhängigkeit vom Abstand der jeweiligen Oberfläche vom Ende eines optischen Wellenleiters für jeweils zwei verschiedene Wellenlängen zeigt;

Fig. 3b)          ein Diagramm, das das jeweilige Verhältnis der Reflexionssignale für die zwei verschiedenen Wellenlängen aus Fig. 3a) zeigt;

Fig. 4a) - e)     weitere Diagramme, die die Abstandsabhängigkeit von Reflexionssignalen von Oberflächen verschiedener Zielobjekte für zwei verschiedene Wellenlängen sowie das Verhältnis der Reflexionssignale für die zwei verschiedenen Wellenlängen zeigen;

Fig. 5            ein weiteres Diagramm, das Verhältnisse von Reflexionssignalen von Oberflächen verschiedener Zielobjekte für zwei verschiedene Wellenlängen in einer praktischen Implementierung des Messverfahrens zeigt;

Fig. 6            eine Prinzipskizze unterschiedlicher Einkopplungen von Messstrahlung in einen optischen Wellenleiter;

Fig. 7            eine weitere Prinzipskizze der Einkopplung von Messstrahlung in einen optischen Wellenleiter;

Fig. 8a) - e)     verschiedene Strahlprofile von aus einem optischen Wellenleiter austretender Messstrahlung, die durch unterschiedliche Einkopplungen der Messstrahlung in den Wellenleiter erzeugt wurden;

Fig. 9            ein Diagramm von Reflexionssignalen für die verschiedenen Strahlprofile in Fig. 8a) bis e) in Abhängigkeit vom Abstand des Endes des Wellenleiters von einer Oberfläche eines Zielobjektes; und

Fig. 10           einen schematischen Aufbau einer Laser-Lithotripsie-Vorrichtung mit der Messvorrichtung in Fig. 1.

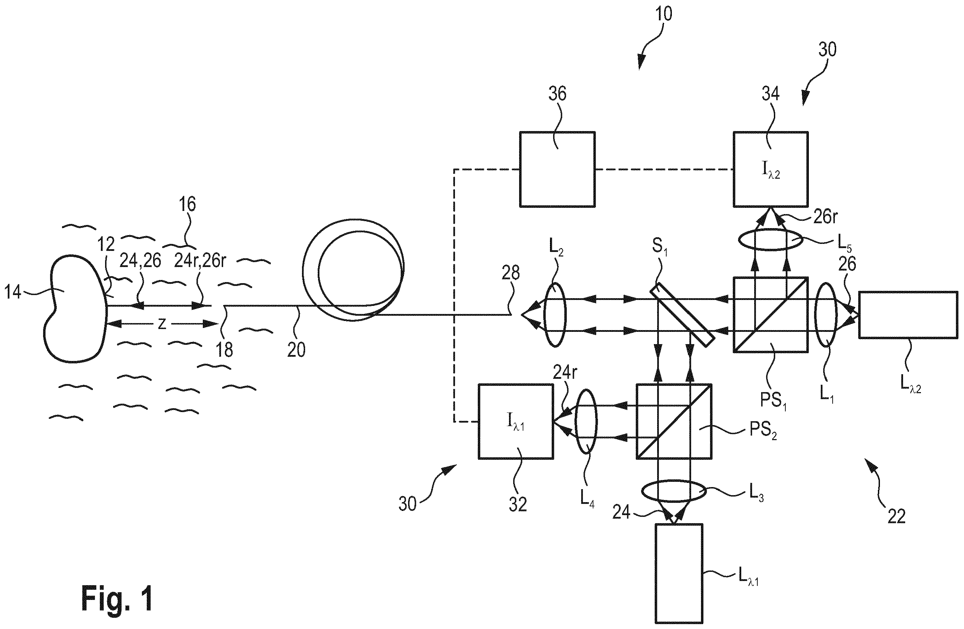

Fig. 1 zeigt ein Ausführungsbeispiel einer Messvorrichtung 10 zum optischen Bestimmen eines Abstandes z einer Oberfläche 12 eines Zielobjektes 14, das sich in einem Medium 16 befindet, von einem Ende 18 eines optischen Wellenleiters 20. Das Medium 16 ist insbesondere ein flüssiges Medium, beispielsweise Wasser oder eine wässrige Lösung. Das Medium 16 kann beispielsweise eine Körperflüssigkeit sein, insbesondere Harnflüssigkeit.

Der optische Wellenleiter 20 kann beispielsweise eine optische Faser, insbesondere eine Multimodenfaser, sein. Der optische Wellenleiter 20 kann auch ein Faserbündel sein.

Die Messvorrichtung 10 weist eine Messstrahlungsquelle 22 zum Erzeugen elektromagnetischer Messstrahlung einer ersten Wellenlänge und einer zweiten Wellenlänge auf. In dem gezeigten Ausführungsbeispiel weist die Messstrahlungsquelle einen ersten Laser $L_{\lambda 1}$ zum Erzeugen elektromagnetischer Messstrahlung 24 einer Wellenlänge $\lambda 1$ und einen zweiten Laser $L_{\lambda 2}$ zum Erzeugen elektromagnetischer Messstrahlung 26 einer Wellenlänge $\lambda 2$ auf, wobei die Wellenlängen $\lambda 1$ und $\lambda 2$ unterschiedlich sind, derart, dass die Messstrahlung der Wellenlänge $\lambda 2$ von dem Medium 16 stärker absorbiert wird als die Messstrahlung der Wellenlänge λ1. In anderen Ausführungsformen können die Messstrahlung 24 und die Messstrahlung 26 auch gemeinsam von einer einzelnen Lichtquelle erzeugt werden, beispielsweise einer breitbandigen Lichtquelle, der ein oder mehrere Spektralfilter nachgeschaltet ist bzw. sind, das bzw. die einen Durchlassbereich bei den Wellenlängen λ1 und λ2 aufweisen.

Die Messstrahlung 24 und die Messstrahlung 26 werden gleichzeitig oder zeitlich geringfügig versetzt in ein Eintrittsende 28 des optischen Wellenleiters 20 eingekoppelt und treten aus dem Ende 18, das das Austrittsende für das Messlicht ist, des Wellenleiters aus.

Die Messvorrichtung 10 kann wie gezeigt mehrere optische Elemente aufweisen. Die Messstrahlung 26, die von dem Laser $L_{\lambda 2}$ erzeugt wird, kann beispielsweise von einer Linse $L_1$ kollimiert werden, durchläuft dann einen Strahlteiler $PS_1$, der ein Polarisationsstrahlteiler sein kann, und einen dichroitischen Spiegel $S_1$ und wird durch eine weitere Linse $L_2$ auf ein dem Ende 18 entgegengesetztes Ende 28 des optischen Wellenleiters 20 gebündelt und in diesen eingekoppelt. Die von dem Laser $L_{\lambda 1}$ erzeugte Messstrahlung 24 wird von einer Linse $L_3$ kollimiert, durchtritt einen Strahlteiler $PS_2$, der ein Polarisationsstrahlteiler sein kann, wird von dem dichroitischen Spiegel $S_1$ zu der Linse $L_2$ umgelenkt und von dieser ebenfalls auf das Ende 28 des optischen Wellenleiters 20 gebündelt und in diesen eingekoppelt.

Die Messvorrichtung 10 weist weiterhin eine Detektionsvorrichtung 30 zum Messen eines ersten Reflexionssignals der von der Oberfläche 12 reflektierten elektromagnetischen Messstrahlung 24r der ersten Wellenlänge λ1 und zum Messen eines zweiten Reflexionssignals der von der Oberfläche 12 reflektierten elektromagnetischen Messstrahlung 26r der zweiten Wellenlänge λ2 auf. Die reflektierte Messstrahlung 24r der ersten Wellenlänge λ1 tritt in das Ende 18 des optischen Wellenleiters 20 ein und aus dem Ende 28 aus, wird von der Linse $L_2$ kollimiert, von dem dichroitischen Spiegel $S_1$ zu dem Strahlteiler $PS_2$ umgelenkt und von diesem zu einer weiteren Linse $L_4$ umgelenkt, die die reflektierte Messstrahlung 24r auf einen ersten Detektor 32 bündelt, um ein erstes Reflexionssignals $I_{\lambda 1}$ zu messen. Die reflektierte Messstrahlung 26r der zweiten Wellenlänge λ2 wird ebenfalls von dem Ende 18 des optischen Wellenleiters 20 eingefangen, tritt aus dem Ende 28 aus, durchläuft die Linse $L_2$ und den dichroitischen Spiegel Si, wird von dem Strahlteiler $PS_1$ zu einer weiteren Linse $L_5$ umgelenkt und von dieser auf einen Detektor 34 gebündelt, um ein zweites Reflexionssignal $I_{\lambda 2}$ zu messen. In anderen Ausführungsformen kann die Detektionsvorrichtung 30 auch nur einen einzelnen Detektor aufweisen, der beide reflektierte Messstrahlungen 24r und 26r empfängt und für beide Wellenlängen λ1 und λ2 sensitiv ist.

Die Messvorrichtung 10 weist weiterhin eine Auswerteeinheit 36 auf, die die Reflexionssignale $I_{\lambda 1}$ und $I_{\lambda 2}$ auswertet, um den Abstand z aus einem Verhältnis der beiden Reflexionssignale $I_{\lambda 2}$ und $I_{\lambda 1}$ zu bestimmen, wie später noch näher beschrieben wird. Die Auswerteeinheit 36 kann auch in die Detektionsvorrichtung 30 integriert sein und kann durch einen Mikroprozessor oder durch Software realisiert sein.

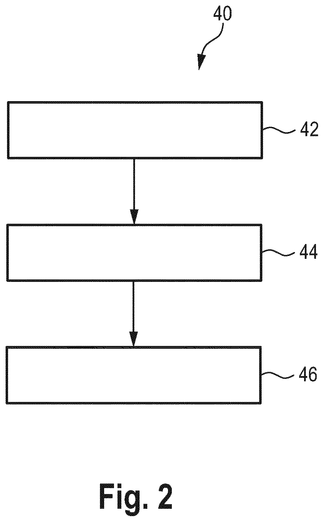

Fig. 2 zeigt ein Flussdiagramm eines Messverfahrens 40 zum optischen Bestimmen eines Abstands z einer in einem Medium 16 befindlichen Oberfläche 12 eines Zielobjekts 14 von einem Ende 18 eines optischen Wellenleiters 20. Das Messverfahren 40 kann mit der Messvorrichtung 10 in Fig. 1 durchgeführt werden, ohne jedoch hierauf beschränkt zu sein.

In einem Schritt 42 wird elektromagnetische Messstrahlung einer ersten Wellenlänge λ1 und einer zweiten Wellenlänge λ2 von dem Ende 18 des Wellenleiters 20 zu der Oberfläche 12 ausgesendet, wobei das Medium 16 die elektromagnetische Messstrahlung der zweiten Wellenlänge λ2 stärker absorbiert als die elektromagnetische Messstrahlung der ersten Wellenlänge λ1. In einem Schritt 44 wird ein erstes Reflexionssignal $I_{\lambda 1}$ der von der Oberfläche 12 reflektierten elektromagnetischen Messstrahlung der ersten Wellenlänge λ1, und ein zweites Reflexionssignal $I_{\lambda 2}$ in der von der Oberfläche 12 reflektierten elektromagnetischen Messstrahlung der zweiten Wellenlänge λ2 gemessen. In einem Schritt 46 wird der Abstand der Oberfläche 12 von dem Ende 18 des optischen Wellenleiters 20 aus einem Verhältnis aus dem zweiten Reflexionssignal $I_{\lambda 2}$ dem ersten Reflexionssignal $I_{\lambda 1}$ bestimmt.

Die Wellenlänge λ1 wird vorzugsweise so gewählt, dass die Absorption des Mediums 16 bei dieser Wellenlänge λ1 nur schwach ist. Die Intensität des Reflexionssignals $I_{\lambda 1}$ bei der Wellenlänge λ1 kann wie folgt beschrieben werden:

$$I_{\lambda 1} = I_{01} \cdot r_1 \cdot d_{\lambda 1}\,(z) \qquad\qquad (1)$$

In Gleichung (1) ist $I_{01}$ das bei Kontakt zwischen dem Ende 18 des optischen Wellenleiters 20 und einer hochreflektierenden Oberfläche (beispielsweise eines Spiegels) gemessene Reflexionssignal (das heißt die maximale messbare Intensität oder das maximal messbare Reflexionssignal), $r_1$ der Reflexionsgrad der Oberfläche 12 und $d_{\lambda 1}$ (z) eine Funktion, die den Abfall des Reflexionssignals mit zunehmendem Abstand z aufgrund der Beleuchtungs- und Detektionsgeometrie beschreibt und von der numerischen Apertur abhängt (wie beispielsweise in dem Artikel von Komives C, Schultz J.S.: "Fiber-Optic Fluorometer Signal Enhancement and Application to Biosensor Design", Talanta 1992, 39(4): 429-441, oder in dem Artikel von V. Svyryd et al.: "An analysis of a displacement sensor based on optical fibers", Revista Meixana de Fisica S 52(2): 61-63 (2006) beschrieben ist).

Da der Reflexionsgrad $r_1$ verschiedener Oberflächen, wie dies bei Oberflächen von Körpersteinen der Fall sein kann, stark variieren kann, kann aus dem Reflexionssignal $I_{\lambda 1}$ nicht auf den Abstand z zwischen dem Ende 18 und der Oberfläche 12 geschlossen werden, selbst wenn $d_{\lambda 1}$ (z) bekannt ist.

Die Wellenlänge λ2 wird vorzugsweise so gewählt, dass die Messstrahlung der Wellenlänge λ2 von dem Medium 16 stärker absorbiert wird als die Messstrahlung der Wellenlänge λ1. Insbesondere wird die Wellenlänge λ2 so gewählt, dass das Reflexionssignal $I_{\lambda 2}$ innerhalb einer gewünschten Distanz $z_{Grenz}$ auf weniger als einen Bruchteil p der maximal messbaren Intensität abfällt. Vorzugsweise wird λ2 so gewählt, dass das zweite Reflexionssignal $I_{\lambda 2}$ in einem Abstand von dem Ende des Wellenleiters unterhalb eines vorbestimmten Maximalabstandes auf einen Bruchteil von weniger als 20%, vorzugsweise von weniger als 10%, weiter vorzugsweise von weniger als 5% der maximal messbaren Intensität $I_{02}$ abfällt.

Für die Wellenlänge λ2 wird die Gleichung (1) dem Lambert-Beerschen Gesetz folgend um einen exponentiellen Faktor erweitert:

$$I_{\lambda 2} = I_{02} \cdot r_2 \cdot d_{\lambda 2} \text{ (z)} \cdot \exp(-2\alpha z) \tag{2}$$

α ist der Absorptionskoeffizient des Mediums 16 bei der Wellenlänge λ2. Der Faktor 2 im Exponenten ergibt sich aus dem zweimaligen Durchlaufen des Abstands z zwischen dem Ende 18 des Wellenleiters 20 und der Oberfläche 12.

Bei gleicher Beleuchtungs- und Detektionsgeometrie bei beiden Wellenlängen λ1 und λ2 ist $d_{\lambda 1}$ (z) ~ $d_{\lambda 2}$ (z). Wird das Verhältnis der beiden Reflexionssignale $I_{\lambda 2}/I_{\lambda 1}$ gebildet, so hängt dieses Verhältnis vom Abstand z gemäß einem exponentiellen Verlauf ab:

$$I_{\lambda 2}/I_{\lambda 1} = (I_{02}/I_{01}) \cdot (r_2/r_1) \cdot \exp(-2\alpha z) \tag{3}$$

Wenn $I_{01}$ und $I_{02}$ für die beiden Wellenlängen λ1 und λ2 gleich oder etwa gleich sind, und auch die Reflexionsgrade $r_1$ und $r_2$ für die beiden Wellenlängen λ1 und λ2 gleich oder näherungsweise gleich sind, reduziert sich die Gleichung (3) auf den Exponentialfaktor. Wenn die Absorption des Mediums 16 bei der Wellenlänge λ2 zumindest näherungsweise bekannt oder bestimmbar ist, kann durch Auflösen der Gleichung (3) nach z kann somit der Abstand z genau bestimmt werden. Die vorstehend genannten Annahmen sind jedoch nicht in allen Fällen gegeben. In praktischen Anwendungen ist es auch nicht erforderlich, den Wert des Abstandes z genau zu kennen, sondern es kann genügen zu detektieren, ob der Abstand z innerhalb eines Abstandsbereichs unterhalb eines vorbestimmten Maximalabstandes und/oder oberhalb eines vorbestimmten Mindestabstandes liegt. Das Messverfahren 40 kann daher so ausgeführt werden, dass bestimmt wird, ob ein vorbestimmter Maximalabstand unterschritten und/oder ein vorbestimmter Mindestabstand überschritten ist, um dann, wenn dies der Fall ist, ein Triggersignal, beispielsweise zum Auslösen eines Vorgangs oder einer Aktion, wie beispielsweise einer Freischaltung einer Vorrichtung, zu erzeugen. Dies wird im folgenden noch beschrieben.

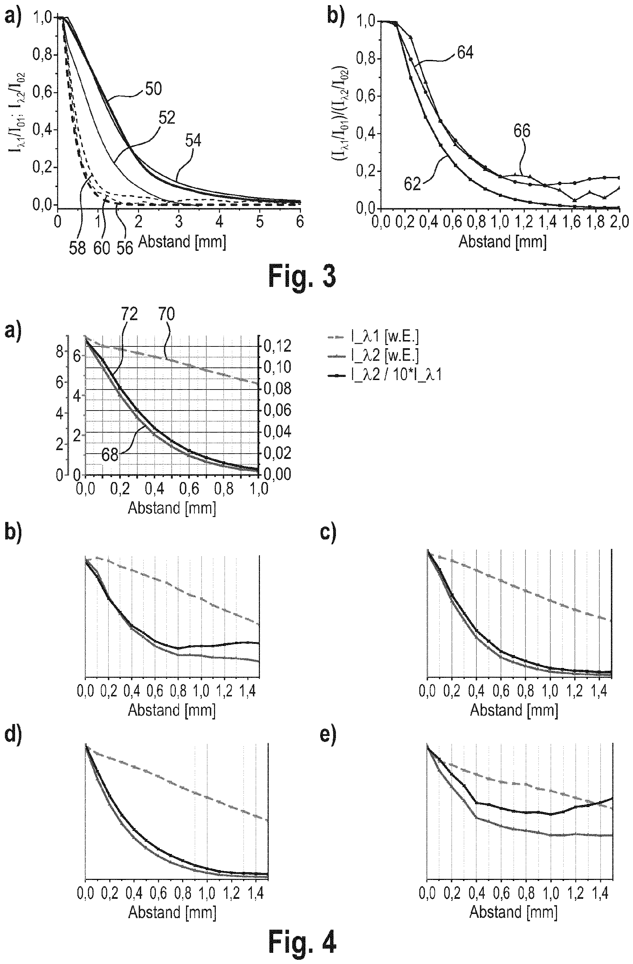

Zur Verifikation der Gleichung (3) wurden Beispielmessungen durchgeführt. Fig. 3a) zeigt ein Diagramm von Kurven aus Beispielmessungen, die mit einer Messvorrichtung ähnlich der Messvorrichtung 10 in Fig. 1 mit elektromagnetischer Messstrahlung einer ersten Wellenlänge λ1 = 520 nm und einer zweiten Wellenlänge λ2 = 1310 nm an einem Spiegel und zwei humanen Steinen als Objekte 14 in Wasser als Medium 16 durchgeführt wurden. Der Absorptionskoeffizient von Wasser bei einer Temperatur von 25 °C beträgt $3{,}035 \times 10^{-5}$ mm$^{-1}$ bei 520 nm und 0,7708 mm$^{-1}$ bei 1310 nm. Die Kurve 50 zeigt die Abstandsabhängigkeit des Reflexionssignals $I_{\lambda 1}$ für den Spiegel, die Kurve 52 den Abstandsverlauf des Reflexionssignals $I_{\lambda 1}$ für den ersten Stein, und die Kurve 54 den Abstandsverlauf des Reflexionssignals $I_{\lambda 1}$ für den zweiten Stein. Die Kurve 56 zeigt den Abstandsverlauf des Reflexionssignals $I_{\lambda 2}$ für den Spiegel, die Kurve 58 den Abstandsverlauf des Reflexionssignals $I_{\lambda 2}$ für den ersten Stein, die Kurve 60 den Abstandsverlauf des Reflexionssignals $I_{\lambda 2}$ für den zweiten Stein. Die Reflexionssignale $I_{\lambda 1}$ sind in Fig. 3a) auf $I_{01}$ normiert, und die Reflexionssignale $I_{\lambda 2}$ auf $I_{02}$. Fig. 3b) zeigt den Abstandsverlauf des Verhältnisses $I_{\lambda 2}/I_{\lambda 1}$ für den Spiegel (Kurve 62), den ersten Stein (Kurve 64) und den zweiten Stein (Kurve 66). $I_{\lambda 1}$ und $I_{\lambda 2}$ sind wiederum auf $I_{01}$ bzw. $I_{02}$ normiert. Die Kurven 56, 58 und 60 in Fig. 3a) fallen im Vergleich zu den Kurven 50, 52, 54 deutlich schneller mit zunehmendem Abstand zwischen dem Ende 18 des Wellenleiters 20 und der Oberfläche 12 des jeweiligen Zielobjektes ab. Aus den Kurven 62, 64 und 66 in Fig. 3b) geht der gemäß Gleichung (3) beschriebene exponentielle Abfall des Verhältnisses $I_{\lambda 2}/I_{\lambda 1}$ hervor. Alle Messkurven wurden auf das jeweilige Maximum normiert. Zu beachten ist hier die Streckung der Abszisse in Fig. 3b) gegenüber der Abszisse in Fig. 3a).

Allgemein kann die zweite Wellenlänge λ2 so gewählt werden, dass sich der Absorptionskoeffizient des Mediums 16 bei der Wellenlänge λ2 von dem Absorptionskoeffizient bei der Wellenlänge λ1 um einen Faktor von zumindest 100, vorzugsweise von zumindest 1000, weiter vorzugsweise von zumindest 10000 unterscheidet. In der Beispielmessung unterscheidet sich der Absorptionskoeffizient von Wasser bei λ2 von dem Absorptionskoeffizient bei λ1 bei einer Temperatur von 25 °C sogar um einen Faktor von über 25.000.

Wenn in der Gleichung (3) die Reflexionsgrade $r_1$, $r_2$ und/oder die maximale Signalintensität $I_{01}$,$I_{02}$ nicht im Wesentlichen gleich und auch nicht bekannt sind, kann es sinnvoll sein, die Wellenlänge $\lambda 2$ so zu wählen, dass Reflexionssignale erst ab Unterschreitung eines vorbestimmten oder gewünschten Maximalabstandes $z_{Grenz}$ gemessen werden können. Dies kann durch einen entsprechend hohen Absorptionskoeffizienten sichergestellt werden. Beispielsweise kann bei einer Wellenlänge $\lambda 2$ = 1310 nm das Reflexionssignal $I_{\lambda 2}$ eines Spiegels in Wasser, je nach numerischer Apertur des Wellenleiters und Einstrahlwinkel auf den Spiegel, innerhalb von 1 mm auf etwa 4 % des Maximalwertes abfallen, wie aus Fig. 4a) aus einer Messkurve 68 für das Reflexionssignal $I_{\lambda 2}$ hervorgeht. Fig. 4a) zeigt beispielhaft den gemessenen Abstandsverlauf des Reflexionssignals $I_{\lambda 1}$ (Kurve 70) für $\lambda 1$=520 nm und den Abstandsverlauf des Verhältnisses $I_{\lambda 2}$/$I_{\lambda 1}$ (Kurve 72) für einen Spiegel. Da die Intensität der Messstrahlung bei der Wellenlänge $\lambda 1$ am Ende 18 des Wellenleiters 20 um einen Faktor 10 niedriger war als bei den anderen Messungen, wurden die Reflexionssignale bei der Wellenlänge $\lambda 1$ entsprechend mit 10 multipliziert. Fig. 4b) bis e) zeigen weitere Beispielmessungen an verschiedenen Körpersteinproben in Wasser/Pufferlösung. Die Messwerte wurden nur untergrundkorrigiert, nicht normiert. In Fig. 4b) bis e) wurden die Messskalen an der Ordinate aus Gründen der Übersichtlichkeit weggelassen. Die Messungen haben jedoch ergeben, dass sich die Absolutwerte der Reflexionssignale der verschiedenen Körpersteine um einen Faktor > 5 sowohl bei der Wellenlänge $\lambda 1$ = 520 nm als auch bei der Wellenlänge $\lambda 2$ = 1310 nm unterscheiden, und dass die Verhältnisse $I_{\lambda 2}$/$I_{\lambda 2}$ im Wesentlichen dem Verlauf der Kurven bei der Wellenlänge $\lambda 2$ folgen, jedoch wertemäßig enger beisammen liegen. Hieraus kann gefolgert werden, dass der Reflexionsgrad $r_1$ bei $\lambda 1$ dem Reflexionsgrad $r_2$ bei $\lambda 2$ für die verschiedenen Körpersteine ausreichend ähnlich ist, um die Reflexionssignale bei $\lambda 2$ mit den Reflexionssignalen bei $\lambda 1$ "normieren" zu können.

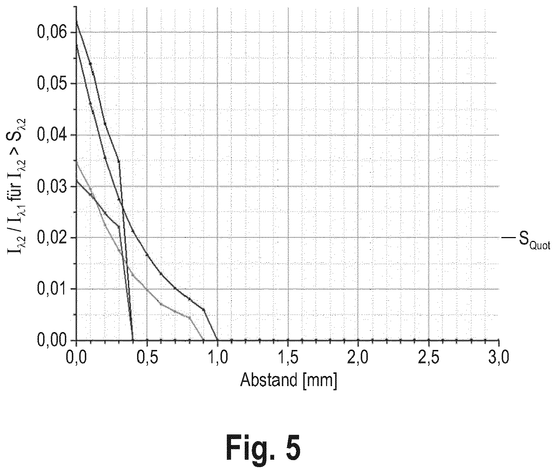

Wenn die Abstandsmessung für den Zweck durchgeführt wird zu detektieren, ob sich das Ende 18 des Wellenleiters in einem Abstand zu der Oberfläche 12 des Zielobjektes befindet, der kleiner als oder allenfalls gleich einem vorbestimmten Maximalabstand $z_{Grenz}$ ist, ist es für die praktische Implementierung des erfindungsgemäßen Messverfahrens vorteilhaft, wenn für das Reflexionssignal $I_{\lambda 2}$ ein Schwellenwert $S_{\lambda 2}$ in Abhängigkeit des vorbestimmten Maximalabstandes $z_{Grenz}$ vorbestimmt wird. Des Weiteren ist es vorteilhaft, auch für das Verhältnis $I_{\lambda 2}$/$I_{\lambda 1}$ einen Schwellenwert $S_{Quot}$ in Abhängigkeit von $z_{Grenz}$ vorzubestimmen. In Fig. 5 ist der Abstandsverlauf des Verhältnisses $I_{\lambda 2}$/$I_{\lambda 1}$ für $I_{\lambda 2}$ > $S_{\lambda 2}$ aus den Kurven in Fig. 4d) bis e) gezeigt. Der für Fig. 5 verwendete Schwellenwert $S_{\lambda 2}$ wurde mit 0,0025 V festgelegt, und die aus Fig. 4b) bis e) entnommenen Kurven für das Verhältnis $I_{\lambda 2}$/$I_{\lambda 1}$ wurden mit einer Treppenfunktion multipliziert, die für $I_{\lambda 2}$ > $S_{\lambda 2}$ 1 ist, und ansonsten 0 ist. Der Schwellenwert $S_{Quot}$ kann beispielsweise auf 0,02 gesetzt werden. Wenn das Messverfahren auf diese Weise bei der Laser-Lithotripsie verwendet wird, bedeutet dies gemäß Fig. 5, dass Körpersteine nur bei einem Abstand < 0,5 mm zwischen dem Ende 18 des Wellenleiters 20 und der Oberfläche 12 des jeweiligen Körpersteine lithotripsiert werden.

Wie aus der vorstehenden Beschreibung hervorgeht, ist es vorteilhaft, wenn das gemessene Reflexionssignal $I_{\lambda 2}$ als Funktion des Abstandes stark abfällt. Eine Änderung des Abstandsverhaltens des Reflexionssignals kann anstelle durch eine Verwendung von langwelliger Messstrahlung, bei der das Medium 16 eine hohe Absorption besitzt, auch durch unterschiedliche Arten der Einkopplung der Messstrahlung in den Wellenleiter 20 erreicht werden, insbesondere durch unterschiedliche Ausnutzung der numerischen Apertur des Wellenleiters 20 und/oder durch eine zur Längsachse des Wellenleiters schräge Einkopplung, mit der Donut-Moden erzeugt werden können.

Wird die Messstrahlung unter einem Öffnungswinkel (Apertur) kleiner als der Akzeptanzwinkel des Wellenleiters 20 in diesen eingekoppelt, ist in der Regel auch der Öffnungswinkel (Apertur) des austretenden Lichtstrahls kleiner und das Reflexionssignal fällt mit zunehmendem Abstand langsamer ab. Wird umgekehrt die Messstrahlung unter einem größeren Öffnungswinkel, beispielsweise gleich dem Akzeptanzwinkel des Wellenleiters 20, in diesen eingekoppelt, ist in der Regel auch der Öffnungswinkel des austretenden Lichtstrahls größer und das Reflexionssignal fällt mit zunehmendem Abstand schneller ab. Bei einer zur Wellenleiterachse schrägen Einkopplung der Messstrahlung 26 entsteht ein Donut-förmiges Strahlprofil, dessen Reflexionssignal sogar noch schneller abfällt. Diese Möglichkeit der Anpassung der Einkopplung kann hilfreich sein, wenn die Messlichtquelle 22 in Fig. 1 keine Messstrahlung mit einer Wellenlänge bereitstellen kann, die von dem Medium 16 im gewünschten Maß absorbiert wird. Prinzipiell können die Wellenlängen $\lambda 1$ und $\lambda 2$ bei dieser Ausführungsform des Messverfahrens gleich oder nahezu gleich sein, wobei die von den Lichtquellen $L_{\lambda 1}$ und $L_{\lambda 2}$ erzeugte jeweilige Messstrahlung lediglich auf unterschiedliche Weise (Öffnungswinkel und/oder Orientierung) in den Wellenleiter 20 eingekoppelt wird.

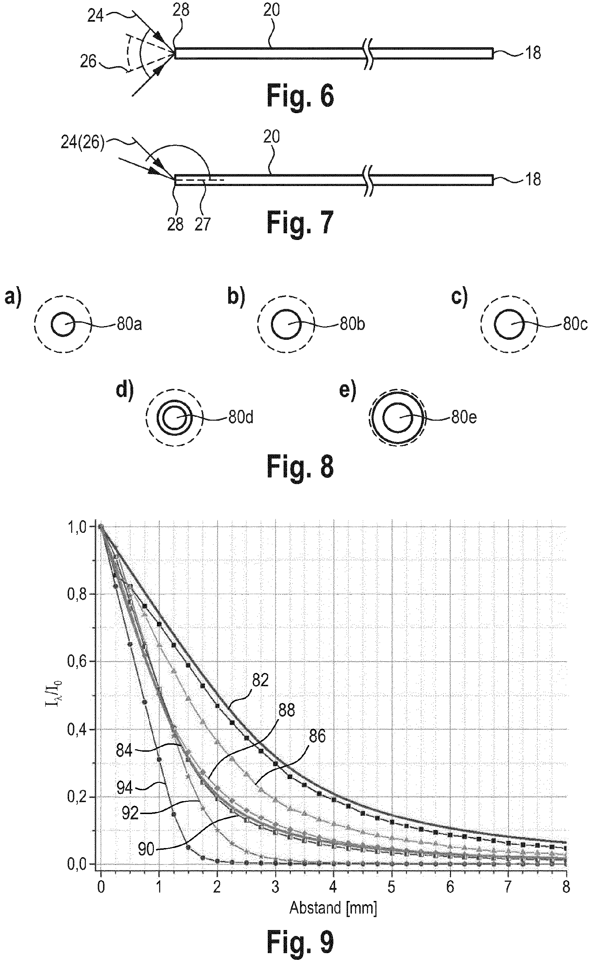

Fig. 6 zeigt die Einkopplung von Messstrahlung 24 mit großem Öffnungswinkel und die Einkopplung von Messstrahlung 26 mit kleinerem Öffnungswinkel, und Fig. 7 zeigt die Einkopplung von Messstrahlung 24 in den Wellenleiter 28 schräg zur Wellenleiterachse 27 des Wellenleiters 20, während die (nicht gezeigte) Messstrahlung 26 beispielsweise kollinear zur Wellenleiterachse 27 eingekoppelt wird.

Fig. 8a) bis e) zeigen unterschiedliche Strahlprofile von Messstrahlung in einem Abstand von 50 mm von dem Ende 18 des Wellenleiters 20. Fig. 8a) zeigt ein Strahlprofil 80a mit kleiner Apertur, Fig. 8b) ein Strahlprofil 80b mit mittlerer Apertur, und Fig. 8c) ein Strahlprofil 80c mit maximaler Apertur. Fig. 8d) zeigt ein Strahlprofil 80b in Form eines Donut-Profils mit kleiner Apertur und Fig. 8e) ein Strahlprofil 80e in Form eines Donut-Profils mit großer Apertur. Die unterbrochenen Kreislinien veranschaulichen die maximal nutzbare Apertur des Wellenleiters 20.

Fig. 9 zeigt das Abstandsverhalten eines Reflexionssignals $I_\lambda$ für verschiedene Arten der Einkopplung von Messstrahlung in den Wellenleiter 20. Die hier betrachtete Wellenlänge beträgt 520 nm, das Medium ist Wasser. Die beiden Kurven ohne Symbole in Fig. 9 zeigen das berechnete Abstandsverhalten des Reflexionssignals $I_\lambda$ für einen Wellenleiter in Form einer Faser mit einem Kerndurchmesser von 365 $\mu$m und einer numerischen Apertur 0,11 (Kurve 82) und einer numerischen Apertur von 0,22 (Kurve 84). Hieran lässt sich erkennen, dass die Wahl eines Wellenleiters mit einer höheren numerischen Apertur zu einem schnelleren Abfall des Reflexionssignals $I_\lambda$ mit zunehmendem Abstand führt. Die Kurven mit Symbolen in Fig. 9 zeigen Messungen an einem Spiegel in Wasser, bei denen die numerische Apertur des Wellenleiters 20 unterschiedlich ausgenutzt bzw. die Messstrahlung schief in den Wellenleiter 20 eingekoppelt wurde. Die Kurve 86 zeigt das Abstandsverhalten des Reflexionssignals $I_\lambda$ für eine Einkopplung der Messstrahlung in den Wellenleiter 20, die das Strahlprofil gemäß Fig. 8a) erzeugt. Die Kurve 88 zeigt das Abstandsverhalten des Reflexionssignals $I_\lambda$ für eine Einkopplung, die das Strahlprofil 80b gemäß Fig. 8b) erzeugt. Die Kurve 90 zeigt das Abstandsverhalten des Reflexionssignals $I_\lambda$ für eine Einkopplung der Messstrahlung, die das Strahlprofil 80c gemäß Fig. 8c) erzeugt. Die Kurve 92 zeigt das Abstandsverhalten des Reflexionssignals $I_\lambda$ für eine Einkopplung, die das Strahlprofil 80d gemäß Fig. 8d) erzeugt. Die Kurve 94 zeigt das Abstandsverhalten des Reflexionssignals $I_\lambda$ für eine Einkopplung, die das Strahlprofil 80e gemäß Fig. 8e) erzeugt. Hieraus ergibt sich, dass mit zunehmender Ausnutzung (bzw. Füllung) der Apertur des Wellenleiters das Reflexionssignal als Funktion des Abstandes zunehmend schneller abfällt, und dass eine schräge Einkopplung, die ein Donut-Strahlprofil erzeugt, ein noch schnelleres Abfallen des Reflexionssignals als Funktion des Abstandes bewirkt.

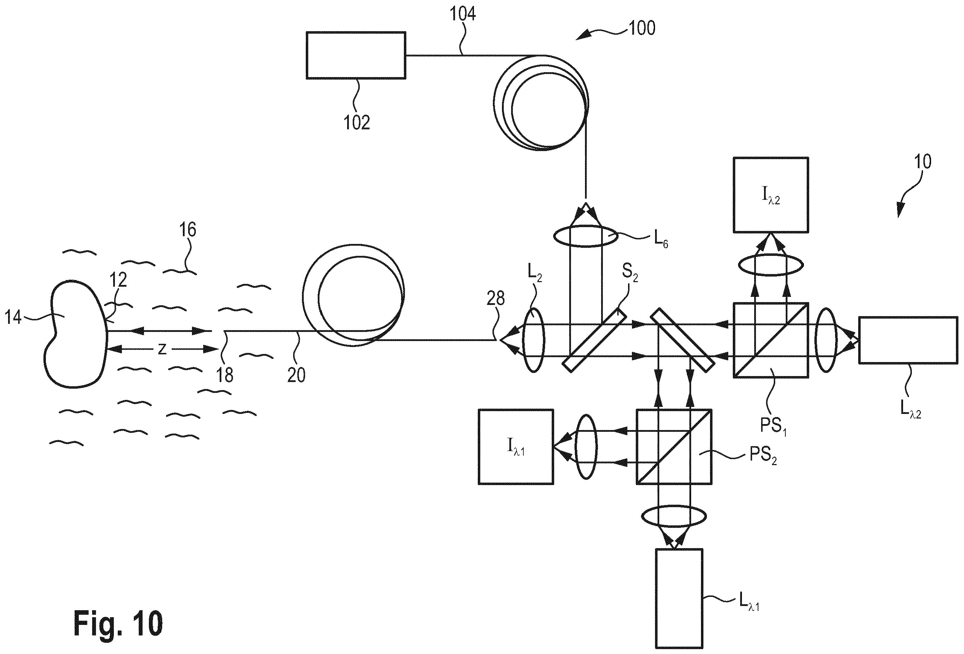

Fig. 10 zeigt ein Blockschaltbild einer Laser-Lithotripsie-Vorrichtung 100, die die Messvorrichtung 10 gemäß Fig. 1 als Bestandteil aufweisen kann. Zusätzlich zu der Messvorrichtung 10 weist die Laser-Lithotripsie-Vorrichtung 100 einen Behandlungslaser 102 auf, der Behandlungslaserlicht zum Zerkleinern von Körpersteinen erzeugt. Der Behandlungslaser 102 kann ein Holmium-Laser sein. Das Behandlungslaserlicht kann über einen Wellenleiter 104, eine weitere Linse $L_6$ und einen weiteren dichroitischen Spiegel $S_2$ und über die Linse $L_2$ in den Wellenleiter 20, der auch für die Messstrahlung genutzt wird, eingekoppelt werden, so dass das Behandlungslaserlicht aus dem Ende 18 des Wellenleiters 20 austreten kann. Der Behandlungslaser 102 kann durch ein Triggersignal der Auswerteeinheit 36 (Fig. 1, in Fig. 10 aus Gründen der Übersichtlichkeit weggelassen) freigeschaltet werden, wenn die Messvorrichtung 10 einen Abstand z des Endes 18 des Wellenleiters 20 von der Oberfläche 12 des Zielobjektes 14 detektiert, der kleiner ist als der Maximalabstand $z_{Grenz}$. Der Maximalabstand $z_{Grenz}$ kann kleiner als 1 mm, beispielsweise kleiner als 0,5 mm sein. Nach Freischaltung kann der Behandlungslaser zur Emission von Behandlungslicht aktiviert werden. Bei der Lithotripsievorrichtung 100 ist somit gewährleistet, dass das Behandlungslaserlicht nur emittiert wird, wenn sich ein Körperstein in einem Abstand vor dem Ende 18 des Wellenleiters 20 befindet, der kleiner ist als der Maximalabstand $z_{Grenz}$. Es kann auch vorteilhaft sein, das Triggersignal nur dann zu erzeugen, wenn der gemessene Abstand des Endes des Wellenleiters 20 von der Oberfläche 12 des Zielobjektes 14 größer als ein Mindestabstand ist, der kleiner ist als der Maximalabstand $z_{Grenz}$. Außerdem kann die Laser-Lithtripsie-Vorrichtung 100 eine Steuerung für den Behandlungslaser 102 aufweisen, die dazu ausgelegt ist, die Pulsenergie des Behandlungslaserlichtes in Abhängigkeit von dem gemessenen Abstand z anzupassen. Beispielsweise kann die Steuerungseinrichtung die Pulsenergie erhöhen, wenn sich das Ende 18 des Wellenleiters 20 in größerem Abstand von der Oberfläche eines zu behandelnden Körpersteines befindet, bzw. bei kleinerem Abstand die Pulsenergie verringern. Die Steuerung kann in den Behandlungslaser oder in die Messvorrichtung 10, beispielsweise in die Auswerteeinrichtung 36, als Hard- oder Software integriert sein.

**Patentansprüche**

1. Messverfahren zum optischen Bestimmen eines Abstands (z) einer in einem Medium (16) befindlichen Oberfläche (12) eines Körpersteins von einem Ende (18) eines optischen Wellenleiters (20), mit den Schritten:

   Aussenden elektromagnetischer Messstrahlung (24, 26) einer ersten Wellenlänge ($\lambda$1) und einer zweiten Wel-lenlänge ($\lambda$2) von dem Ende (18) des Wellenleiters (20) zu der Oberfläche (12), wobei das Medium (16) die elektromagnetische Messstrahlung (26) der zweiten Wellenlänge ($\lambda$2) stärker absorbiert als die elektromagnetische Messstrahlung (24) der ersten Wellenlänge ($\lambda$1);
   Messen eines ersten Reflexionssignals ($I_1$) der von der Oberfläche (12) reflektierten elektromagnetischen Messstrahlung (24r) der ersten Wellenlänge ($\lambda$1), und Messen eines zweiten Reflexionssignals ($I_2$) der von der Oberfläche (12) reflektierten elektromagnetischen Messstrahlung (26r) der zweiten Wellenlänge ($\lambda$2), und Bestimmen des Abstandes (z) aus einem Verhältnis ($I_2/I_1$) aus dem zweiten und dem ersten Reflexionssignal.

2. Messverfahren nach Anspruch 1, wobei das Bestimmen des Abstandes (z) ein Bestimmen umfasst, ob ein vorbestimmter Maximalabstand ($z_{Grenz}$) unterschritten und/oder ein vorbestimmter Mindestabstand überschritten ist.

3. Messverfahren nach Anspruch 2, wobei ein Triggersignal erzeugt wird, wenn die Messung ergibt, dass der vorbestimmte Maximalabstand ($z_{Grenz}$) unterschritten und/oder der vorbestimmte Mindestabstand überschritten ist.

4. Messverfahren nach Anspruch 2 oder 3, wobei die zweite Wellenlänge ($\lambda 2$) so gewählt wird, dass das zweite Reflexionssignal ($I_2$) in einem Abstand von dem Ende (18) des Wellenleiters (20) unterhalb des vorbestimmten Maximalabstandes ($z_{Grenz}$) auf einen Bruchteil von weniger als 20%, vorzugsweise von weniger als 10%, weiter vorzugsweise von weniger als 5% der maximal messbaren Intensität ($I_{02}$) abfällt.

5. Messverfahren nach einem der Ansprüche 2 bis 4, wobei in Abhängigkeit des vorbestimmten Maximalabstandes ($z_{Grenz}$) ein erster Schwellenwert ($S_{\lambda 2}$) für das zweite Reflexionssignal ($I_2$) vorbestimmt wird, und wobei das Verhältnis ($I_2/I_1$) aus dem zweiten Reflexionssignal und dem ersten Reflexionssignal mit 1 multipliziert wird, wenn das zweite Reflexionssignal ($I_2$) den Schwellenwert ($S_{\lambda 2}$) für das zweite Reflexionssignal ($I_2$) überschreitet, und ansonsten auf null gesetzt wird, und wobei in Abhängigkeit des vorbestimmten Maximalabstandes ($z_{Grenz}$) ein zweiter Schwellenwert ($S_{Quot}$) für das Verhältnis ($I_2/I_1$) aus dem zweiten Reflexionssignal und dem ersten Reflexionssignal vorbestimmt wird.

6. Messverfahren nach Anspruch 3 und 5, wobei das Triggersignal erzeugt wird, wenn das Verhältnis ($I_2/I_1$) aus dem zweiten Reflexionssignal und dem ersten Reflexionssignal den zweiten Schwellenwert ($S_{Quot}$) überschreitet.

7. Messverfahren nach einem der Ansprüche 1 bis 6, wobei die zweite Wellenlänge ($\lambda 2$) so gewählt wird, dass sich der Absorptionskoeffizient (a) des Mediums (16) bei der zweiten Wellenlänge ($\lambda 2$) von dem Absorptionskoeffizient bei der ersten Wellenlänge ($\lambda 1$) um einen Faktor von zumindest 100, vorzugsweise von zumindest 1000, weiter vorzugsweise von zumindest 10000 unterscheidet.

8. Messverfahren nach einem der Ansprüche 1 bis 7, wobei die erste Wellenlänge ($\lambda 1$) im sichtbaren Spektralbereich liegt.

9. Messverfahren nach einem der Ansprüche 1 bis 8, wobei die zweite Wellenlänge ($\lambda 2$) im Nah-Infrarot-Spektralbereich liegt.

10. Messverfahren nach einem der Ansprüche 1 bis 9, wobei die Messstrahlung (24) der ersten Wellenlänge ($\lambda 1$) mit einem ersten Öffnungswinkel in den Wellenleiter (20) eingekoppelt wird, der sich von einem Öffnungswinkel unterscheidet, mit dem die Messstrahlung der zweiten Wellenlänge ($\lambda 2$) in den Wellenleiter (20) eingekoppelt wird.

11. Messverfahren nach einem der Ansprüche 1 bis 10, wobei die Messstrahlung (24) der ersten Wellenlänge ($\lambda 1$) oder die Messstrahlung (26) der zweiten Wellenlänge ($\lambda 2$) schräg zur Wellenleiterachse (27) in den Wellenleiter (20) eingekoppelt wird.

12. Messvorrichtung zum optischen Bestimmen eines Abstands (z) einer in einem Medium (16) befindlichen Oberfläche (12) eines Körpersteins von einem Ende (18) eines optischen Wellenleiters (20), mit:

   einer Messstrahlungsquelle (22) zum Erzeugen elektromagnetischer Messstrahlung (24) einer ersten Wellenlänge ($\lambda 1$) und einer zweiten Wellenlänge ($\lambda 2$), wobei das Medium (16) die elektromagnetische Messstrahlung (26) der zweiten Wellenlänge ($\lambda 2$) stärker absorbiert als die elektromagnetische Messstrahlung (24) der ersten Wellenlänge ($\lambda 1$),
   dem optischen Wellenleiter (20) zum Aussenden der elektromagnetischen Messstrahlung (24, 26) von dem Ende (18) des Wellenleiters (20) zu der Oberfläche (12);
   einer Detektionsvorrichtung (30) zum Messen eines ersten Reflexionssignals ($I_1$) der von der Oberfläche (12) reflektierten elektromagnetischen Messstrahlung (24r) der ersten Wellenlänge ($\lambda 1$), und zum Messen eines zweiten Reflexionssignals ($I_2$) der von der Oberfläche (12) reflektierten elektromagnetischen Messstrahlung (26r) der zweiten Wellenlänge ($\lambda 2$), und
   einer Auswerteeinheit (36) zum Bestimmen des Abstandes (z) aus einem Verhältnis ($I_2/I_1$) aus dem zweiten und dem ersten Reflexionssignal.

13. Messvorrichtung nach Anspruch 12, wobei die Messstrahlungsquelle (22) einen ersten Laser ($L_{\lambda 1}$) zum Erzeugen der elektromagnetischen Messstrahlung (24) der ersten Wellenlänge ($\lambda 1$) und einen zweiten Laser ($L_{\lambda 2}$) zum Erzeugen der elektromagnetischen Messstrahlung (26) der zweiten Wellenlänge ($\lambda 2$) aufweist.

**14.** Messvorrichtung nach Anspruch 12 oder 13, wobei der optische Wellenleiter (20) eine numerische Apertur von größer als 0,1, vorzugsweise größer als 0,2 aufweist.

**15.** Laser-Lithotripsie-Vorrichtung zum Zerkleinern von Körpersteinen, mit einem Behandlungslaser (102) zum Emittieren von Behandlungslaserlicht und einer Messvorrichtung (10) nach einem der Ansprüche 12 bis 14.

**16.** Laser-Lithotripsie-Vorrichtung nach Anspruch 15, wobei die Auswerteeinheit (36) der Messvorrichtung (10) dazu ausgelegt ist, ein Triggersignal zum Freischalten des Behandlungslasers (102) zu erzeugen, wenn ein Abstand (z) des Endes (18) des Wellenleiters (20) von der Oberfläche (12) eines zu zerkleinernden Körpersteins gemessen wird, der kleiner ist als ein vorbestimmter Maximalabstand ($z_{Grenz}$).

**17.** Laser-Lithotripsie-Vorrichtung nach Anspruch 16, wobei das Triggersignal erzeugt wird, wenn der gemessene Abstand (z) des Endes (18) des Wellenleiters (20) von der Oberfläche (12) größer als ein Mindestabstand ist, der kleiner ist als der Maximalabstand.

**18.** Laser-Lithotripsie-Vorrichtung nach einem der Ansprüche 15 bis 17, wobei eine Steuerungseinrichtung für den Behandlungslaser (102) dazu ausgelegt ist, die Pulsenergie des Behandlungslaserlichtes in Abhängigkeit von dem gemessenen Abstand (z) anzupassen.

Fig. 1

Fig. 2

Fig. 3

Fig. 4

Fig. 5

Fig. 6

Fig. 7

Fig. 8

Fig. 9

Fig. 10

EP 4 088 677 A1

Europäisches
Patentamt

European
Patent Office

Office européen
des brevets

**EUROPÄISCHER RECHERCHENBERICHT**

Nummer der Anmeldung

EP 22 17 1604

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,P | WO 2021/144801 A1 (LUMENIS LTD [IL]) 22. Juli 2021 (2021-07-22) <br><br> * Absatz [0092] – Absatz [0093] * <br> * Absatz [0107]; Abbildung 2a * <br> * Absatz [0133] – Absatz [0142]; Abbildung 3 * <br> * Absatz [0166] – Absatz [0168] * <br> * Absatz [0131]; Abbildung 2g * <br> ----- | 1-3,9, 11-13, 15,16,18 | INV. A61B18/26 G01B11/02 <br><br> ADD. A61B90/00 A61B18/00 |
| E | WO 2022/112977 A1 (LUMENIS LTD [IL]) 2. Juni 2022 (2022-06-02) <br><br> * Absatz [0047] – Absatz [0058]; Abbildung 2a * <br> * Absatz [0099]; Abbildung 2h * <br> * Absatz [0102] – Absatz [0140]; Abbildungen 3a,b * <br> * Absatz [0155] * <br> ----- | 1-3,9, 11-13, 15,16,18 | |
| Y | WO 2021/026161 A1 (GYRUS ACMI INC D B A OLYMPUS SURGICAL TECH AMERICA [US]) 11. Februar 2021 (2021-02-11) <br> * Absatz [0068]; Abbildung 1 * <br> * Absatz [0115]; Abbildung 10A * <br> * Absatz [0136] – Absatz [0146]; Abbildungen 22a-d * <br> ----- | 1-3, 7-13, 15-18 | RECHERCHIERTE SACHGEBIETE (IPC) <br><br> A61B G01B |
| Y | US 2016/178508 A1 (RAMSTEINER INGO [DE]) 23. Juni 2016 (2016-06-23) <br><br> * Absatz [0040] – Absatz [0045]; Abbildung 9 * <br> * Absatz [0048] * <br> ----- <br><br> -/-- | 1-3, 9-13, 15-18 | |

Der vorliegende Recherchenbericht wurde für alle Patentansprüche erstellt

| Recherchenort | Abschlußdatum der Recherche | Prüfer |
|---|---|---|
| Den Haag | 29. September 2022 | Ekstrand, Vilhelm |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.............................................................
& : Mitglied der gleichen Patentfamilie, übereinstimmendes Dokument

EPO FORM 1503 03.82 (P04C03)

**Seite 1 von 2**

Europäisches
Patentamt

European
Patent Office

Office européen
des brevets

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 84/04439 A1 (CENTRAL ELECTR GENERAT BOARD [GB]) 8. November 1984 (1984-11-08) * Seite 28, Zeile 14 - Seite 33, Zeile 21; Abbildungen 12-14 * ----- | 7,8 | |
| A | EP 3 725 213 A1 (BOSTON SCIENT SCIMED INC [US]) 21. Oktober 2020 (2020-10-21) * Absatz [0177] - Absatz [0178] * ----- | 1-18 | |
| A | WO 2020/174686 A1 (OLYMPUS CORP [JP]) 3. September 2020 (2020-09-03) * Zusammenfassung * ----- | 1-18 | |
| A | US 2013/123769 A1 (KHATCHATUROV ARKADY [IL] ET AL) 16. Mai 2013 (2013-05-16) * Absatz [0042] * ----- | 1-18 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 29. September 2022 | Ekstrand, Vilhelm |

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 22 17 1604

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-09-2022

| Im Recherchenbericht angeführtes Patentdokument | Datum der Veröffentlichung | Mitglied(er) der Patentfamilie | Datum der Veröffentlichung |
|---|---|---|---|
| WO 2021144801 A1 | 22-07-2021 | AU 2021207767 A1 | 14-07-2022 |
| | | CN 114980831 A | 30-08-2022 |
| | | EP 4069125 A1 | 12-10-2022 |
| | | IL 294550 A | 01-09-2022 |
| | | WO 2021144801 A1 | 22-07-2021 |
| WO 2022112977 A1 | 02-06-2022 | US 2022160435 A1 | 26-05-2022 |
| | | US 2022166178 A1 | 26-05-2022 |
| | | WO 2022112977 A1 | 02-06-2022 |
| | | WO 2022112978 A1 | 02-06-2022 |
| WO 2021026161 A1 | 11-02-2021 | CN 114449946 A | 06-05-2022 |
| | | DE 112020003715 T5 | 21-04-2022 |
| | | US 2021038306 A1 | 11-02-2021 |
| | | WO 2021026161 A1 | 11-02-2021 |
| US 2016178508 A1 | 23-06-2016 | CN 105823732 A | 03-08-2016 |
| | | DE 102014226827 A1 | 23-06-2016 |
| | | US 2016178508 A1 | 23-06-2016 |
| WO 8404439 A1 | 08-11-1984 | EP 0153924 A1 | 11-09-1985 |
| | | GB 2150689 A | 03-07-1985 |
| | | JP S61501587 A | 31-07-1986 |
| | | US 4713538 A | 15-12-1987 |
| | | WO 8404439 A1 | 08-11-1984 |
| EP 3725213 A1 | 21-10-2020 | AU 2016274690 A1 | 07-12-2017 |
| | | AU 2020220085 A1 | 03-09-2020 |
| | | CA 2988519 A1 | 15-12-2016 |
| | | CN 107743376 A | 27-02-2018 |
| | | EP 3307138 A1 | 18-04-2018 |
| | | EP 3725213 A1 | 21-10-2020 |
| | | JP 6842431 B2 | 17-03-2021 |
| | | JP 2018516705 A | 28-06-2018 |
| | | US 2016361120 A1 | 15-12-2016 |
| | | US 2020253665 A1 | 13-08-2020 |
| | | WO 2016201092 A1 | 15-12-2016 |
| WO 2020174686 A1 | 03-09-2020 | CN 113490463 A | 08-10-2021 |
| | | JP WO2020174686 A1 | 16-12-2021 |
| | | US 2021378745 A1 | 09-12-2021 |
| | | WO 2020174686 A1 | 03-09-2020 |
| US 2013123769 A1 | 16-05-2013 | CA 2843246 A1 | 02-02-2012 |
| | | EP 2598073 A1 | 05-06-2013 |
| | | IL 224281 A | 29-09-2016 |

Für nähere Einzelheiten zu diesem Anhang : siehe Amtsblatt des Europäischen Patentamts, Nr.12/82

EPO FORM P0461

Seite 1 von 2

**ANHANG ZUM EUROPÄISCHEN RECHERCHENBERICHT
ÜBER DIE EUROPÄISCHE PATENTANMELDUNG NR.**

EP 22 17 1604

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-09-2022

| Im Recherchenbericht angeführtes Patentdokument | Datum der Veröffentlichung | Mitglied(er) der Patentfamilie | | Datum der Veröffentlichung |
|---|---|---|---|---|
| | | US | 2013123769 A1 | 16-05-2013 |
| | | WO | 2012014145 A1 | 02-02-2012 |

Für nähere Einzelheiten zu diesem Anhang : siehe Amtsblatt des Europäischen Patentamts, Nr.12/82

EPO FORM P0461

Seite 2 von 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **LANGE B. ; CORDES J. ; BRINKMANN R.** Stone/tissue differentiation for holmium laser lithotripsy using autofluorescence. *Lasers Surg Med,* 2015, vol. 47 (9), 737-744
- **SCHLAGER D. ; MIERNIK A. ; LAMRINI S. et al.** A novel laser lithotripsy system with automatic real-time urinary stone recognition: Computer controlled ex vivo lithotripsy is feasible and reproducible in endoscopic stone fragmentation. *Journal of Urology,* 2019, vol. 202, 1263-1269

- **LANGE B. ; CORDES J. ; BRINKMANN R.** Exploiting the aiming beam to increase the safety of laser lithotripsy: Experimental evaluation of light reflection and fluorescence. *Lasers Surg Med,* 2020, vol. 52 (5), 456-471
- **KOMIVES C ; SCHULTZ J.S.** Fiber-Optic Fluorometer Signal Enhancement and Application to Biosensor Design. *Talanta,* 1992, vol. 39 (4), 429-441
- **V. SVYRYD et al.** An analysis of a displacement sensor based on optical fibers. *Revista Meixana de Fisica S,* 2006, vol. 52 (2), 61-63